(19) Europäisches Patentamt
European Patent Office
Office européen des brevets

(11) Publication number: 0 260 151 A1

(12) # EUROPEAN PATENT APPLICATION

(21) Application number: 87308069.1

(22) Date of filing: 11.09.87

(51) Int. Cl.$^4$: C 07 C 15/16
C 07 C 2/84

(30) Priority: 12.09.86 US 906472

(43) Date of publication of application: 16.03.88 Bulletin 88/11

(84) Designated Contracting States: AT BE CH DE ES FR GB IT LI NL SE

(71) Applicant: THE DOW CHEMICAL COMPANY
2030 Dow Center Abbott Road P.O. Box 1967
Midland, MI 48640 (US)

(72) Inventor: King, Stanley S. T.
1311 Kirkland Drive
Midland Michigan 48640 (US)

(74) Representative: Burford, Anthony Frederick et al
W.H. Beck, Greener & Co. 7 Stone Buildings Lincoln's Inn
London WC2A 3SZ (GB)

(54) **Oxidative coupling with methyl-substituted benzenes.**

(57) (Methyl-substituted) diphenyl methanes are prepared by coupling at least a methyl-substituted benzene and optionally benzene into the (methyl-substituted) diphenyl methane by contacting at least the methyl-substituted benzene with a solid heterogeneous reactant-catalyst having labile oxygen under conditions whereby the (methyl-substituted) diphenyl methane is prepared. The solid heterogeneous reactant-catalyst having labile oxygen is selected from a vanadium oxide, a molybdenum oxide, a rhenium oxide, a tungsten oxide and other oxide catalysts. By-product formation, especially formation of carbon dioxide, can be very low.

EP 0 260 151 A1

**Description**

OXIDATIVE COUPLING WITH METHYL-SUBSTITUTED BENZENES

This invention concerns a process for preparing coupled aromatic compounds.

The catalytic oxidation of toluene can produce benzaldehyde and benzoic acid. See, for example, U.S. Patent 4,390,728 (1983); Chem. Abs., 97(15):126,725m, abstracting Madhock et al., Indian J. Technol., 20(5), 184-89 (1982); and Anderson, J. Catal., 98, 138 (1986).

Anthraquinone has been allegedly prepared by the use of diluted toluene in air (1.2 percent). See, for example, Japan Kokai Nos. 1982-24323 and 1983-121238. Such known processes to prepare anthraquinone by direct toluene oxidation can be generally inefficient. Loss of valuable reactant materials through complete combustion to carbon dioxide can be particularly problematical.

The present invention is a process for preparing a (methyl-substituted)diphenyl methane by a coupling reaction which process comprises reacting a methyl-substituted benzene with a solid heterogeneous reactant-catalyst having labile oxygen under conditions whereby the (methyl-substituted)diphenyl methane is prepared, said solid heterogeneous reactant-catalyst being a compound having labile oxygen being selected from the group consisting of a vanadium oxide, a molybdenum oxide, a rhenium oxide, a tungsten oxide, a bismuthmolybdate-containing catalyst, and a compound of the formula $M^1_aM^2_bM^3_cO_x$ defined hereinbelow.

The present process is highly efficient. By-product formation, and loss of reactants such as by carbon dioxide formation, is greatly minimized. The process can be carried out in either vapor or liquid phases.

The process prepares useful products. The (methyl-substituted)diphenyl methanes are generally useful as chemical intermediates for preparing anthraquinones which are generally useful, for example, as dye intermediates and pulping catalysts.

In general, the methyl-substituted benzenes include compounds represented by the formula

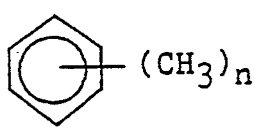

(I)

wherein n is an integer from 1 to 6, preferably from 1 to 5, more preferably from 1 to 3, most preferably 1 or 2. Especially preferred of the methyl-substituted benzenes are toluene and/or xylene. At least one of the methyl-substituted benzenes to be coupled into the methyl-substituted diphenyl methanes have at least one hydrogen available on the benzene ring.

Benzene itself can be employed as reactant for coupling by the process of the invention, so long as the methyl-substituted benzene is also present as reactant. Hence, "at least" may modify methyl-substituted benzene. However, the resulting (methyl-substituted)diphenyl methane is, of course, to a larger extent, a mixture of coupled products, one of which can be diphenyl methane.

In general, the (methyl-substituted)diphenyl methanes are coupled compounds represented by the formula

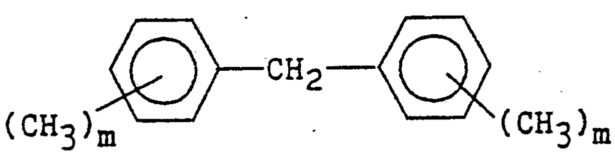

(II)

wherein m is separately at each occurrence an integer from zero to 5, preferably zero to 4, more preferably from zero to 2, most preferably zero or one, and especially one m value is zero while the other m value is one. Preferably, at least one methyl group(s) of the compounds of the formula (II) is (are) in a position ortho to the coupled methylene group such as found, for example, in (2-methylphenyl)phenylmethane.

The reactant-catalyst employed in the process of the invention is a heterogeneous solid. The solid heterogeneous reactant-catalyst contains labile oxygen. The term "reactant-catalyst" means a substance which generally can be considered both or either a reactant and/or a catalyst. As a reactant, the heterogeneous substance generally supplies the labile oxygen to the remaining components of the reaction medium, itself thus losing the oxygen. As a catalyst, the heterogeneous substance generally can be regenerated in the catalytic cycle. The term "labile", which modifies oxygen, means able to be supplied to the remaining components of the reaction medium from the heterogeneous reactant-catalyst. For example, some of the suitable compounds for the reactant-catalyst may be represented by the following formula

$M^1_aM^2_bM^3_cO_x$ (III)

wherein $M^1$ is hydrogen;

$M^2$ is other elements;

$M^3$ is at least one of V, Mo, Re, La or W;

a is 0 to 10;

b is 0 to 100;

c is 0.01 to 100; and
x is a number which satisfies the average valances of $M^1$, $M^2$ and $M^3$ in the oxidation states in which they exist in reactant-catalyst.
The reactant-catalyst of the invention may be supported with a support such as, for example, silica or alumina.

For example, toluene reacts with oxygen in metal oxides such as, in general, vanadium oxide, e.g. $V_2O_5$, molybdenum oxide, e.g. $MoO_3$, rhenium oxide, e.g. $Re_2O_7$, tungsten oxide, e.g. $WO_3$, bismuthmolybdate-containing catalysts such as, for example, heteropoly(cage)molybdates and/or tungstates, without gas phase oxygen to form (2-methylphenyl)phenylmethane at 150 to 500°C; and thus, substances such as these are solid heterogeneous reactant-catalysts having labile oxygen under these general conditions. The bismuthmolybdate-containing catalyst is such as disclosed in U.S. Patent 2,904,580 (1957) or more generally termed, a bismuth phosphomolybdate catalyst. The most preferred species of the solid heterogeneous reactant-catalysts are, in general, $MoO_3$ and $V_2O_5$.

In general, the temperatures of the process are elevated. Elevated temperatures include those such as from 50 to 700°C. Preferred elevated temperatures generally range from 100 to 500°C. More preferable elevated temperatures for the vapor phase process are generally from 200 to 450°C. Elevated temperatures of the process carried out in the liquid phase may be generally lower than those of the process carried out in the vapor phase. Preferred elevated temperatures can vary with the specific solid heterogeneous reactant-catalyst employed such as illustrated for the vapor phase process by the most preferred temperatures of the following table.

| Catalyst | General Temperature |
|---|---|
| Generally, $V_2O_5$ | 350°C |
| Generally, $MoO_3$ or Type SA | 400°C |

In general, the liquid phase process is carried out neat, and the vapor phase process can be carried out near or with an inert gaseous diluent present as a so-called carrier gas. Preferred carrier gases include nitrogen, helium and argon. Unreacted methyl-substituted benzenes are preferably recycled as a reactant.

To couple the methyl-substituted benzenes into the (methyl-substituted)diphenyl methanes, at least the methyl-substituted benzenes are brought into contact with the solid heterogeneous reactant-catalyst having labile oxygen. Oxygen such as gaseous oxygen is preferably absent during the coupling or present in less than the stoichiometric amount. The (methyl-substituted)diphenyl methanes are prepared, and the available labile oxygen of the solid heterogeneous reactant-catalyst depletes.

Generally, subsequent to the depletion of the labile oxygen of the solid heterogeneous reactant-catalyst, the reactant-catalyst is regenerated by contact with oxygen to provide available labile oxygen to the solid heterogeneous reactant-catalyst. The regenerative contact is generally with oxygen, for example, gaseous oxygen, including gaseous oxygen dissolved in at least one carrier gas, for example, as in air. The regenerative contact with the oxygen need not be direct as in air contacting the same general site of the prior contact of at least the methyl-substituted benzenes with the now depleted solid heterogeneous reactant-catalyst itself, but can be by a more circuitous route such as by diffusion through a solid such as by air contacting the "back side" of the solid heterogeneous reactant-catalyst or membrane upon which it can be supported, which is not the same general site as of the prior, or even ongoing, contact of the methyl-substituted benzene(s) with the solid heterogeneous reactant-catalyst.

Preferably, the replenishing of the labile oxygen is by air. Multiple alternating beds of solid heterogeneous reactant-catalyst at various stages of coupling and depletion of labile oxygen and its regeneration can be employed.

The (methyl-substituted)diphenyl methanes can be recovered, and/or purified if desired, by known procedures. Preferred are low temperature vapor traps.

Conversion herein is the molar percent of the organic reactant(s) which is (are) changed into any and all product(s). The conversion can vary widely depending on operating conditions, and can typically be from one to 80 mole percent. Selectivity herein is the molar percent of a specific product(s) which is (are) prepared, based upon moles of the organic reactant(s) which is (are) converted, that is, based upon the conversion. For example, if 10 moles of the organic reactant toluene are employed and 5 moles of the toluene are changed into any and all product(s), then the conversion is 50 percent. If, of the 5 moles of the toluene which are converted, one mole of the toluene appears as 7 moles of carbon dioxide, then selectivity to carbon dioxide is 20 percent. If, of the 5 moles of the toluene which are converted, 3 moles of the toluene appears as 1.5 moles of a (methylphenyl)phenylmethane, then selectivity to (methylphenyl)phenylmethane(s) is 60 percent. Preferably, selectivity to the (methyl-substituted)diphenyl methane is at least 20 percent, more preferably at least 40 percent and most preferably at least 60 percent. Concurrently thus, by-product formation can be correspondingly low. Especially notable is the extremely low level of formation of the by-product carbon dioxide which is preferably formed at the selectivity of at most about 65 percent, more preferably at most 30 percent and most preferably at most 10 percent.

The following examples further illustrate the invention. Parts, percentages and ratios are by weight unless otherwise specified.

In the following Examples 1-2, the reactant-catalyst was packed in a reactor and heated to the desired temperature. The methyl-substituted benzene was fed to the reactor by an inert carrier gas. The products and the unreacted methyl-substituted benzenes were collected at the exit of the reactor. The reactor bed was then purged and regenerated by passing the oxygen-containing gas (e.g. air) through the reactor.

Example 1 - Vapor Phase

$MoO_3$ powder (0.109 g) was heated in a quartz tube to 400°C with helium purge at a constant rate of 30 cc/min. Toluene was introduced into the reactor in the helium stream at a rate of 50 µl/min. The reaction products were analyzed by an on-line gas chromatograph and an infrared spectrometer, and 70 percent of the product was two methyldiphenylmethane (MDPM) isomers. The other 30 percent contains benzaldehyde, methylbenzophenones, anthraquinone and a small amount of carbon dioxide. The catalyst deactivates in 5 minutes due to the depletion of the oxygen. 0.47 mg ot MDPM isomers were obtained. Upon purging of the toluene from the reactor, air was used to regenerate the catalyst. A total of 55 micromoles of carbon dioxide was collected from the combustion of coke on catalyst. The toluene selectivities to MDPM, coke and others such as benzaldehyde, benzoic acid and benzophenone were 34 percent, 52 percent and 14 percent, respectively.

Example 2 - Vapor Phase

Similar to the procedure of Example 1, ortho-xylene was employed as the organic reactant. Gas chromatography analysis showed production of (methyl-substituted)diphenyl methanes.

Example 3 - Liquid Phase

A sample of 0.57 g of toluene was heated with 0.52 g of $V_2O_5$ at 200°C in a small stainless steel container for 2 hours. One percent of the toluene was converted into MDPM, anthraquinone, benzaldehyde and carbon dioxide at respective selectivities of 70.3 percent, 18.7 percent, 8.2 percent and 2.8 percent.

In the following Examples 4-19, the methyl-substituted benzenes and the reactant catalyst were mixed in a sealed pressure reactor. The reactor was heated to the desired temperature for the desired time. After the reaction, the products and the unreacted methyl-substituted benzenes were separated from the solid. The desired products were purified from the mixture. The spent reactant-catalyst was regenerated by heating with oxygen containing gas (e.g. air). The temperature range used was 50 to 700°C and the heating time used from 1 min. to 24 hrs.

Example 4

Toluene (0.43 g) and $V_2O_5$ (0.31 g) were heated in a two cc stainless steel reactor to 300°C for 3 hours. After the reactor was cooled to room temperature the liquid content was analyzed by gas chromatography. Ten percent of the toluene was converted and the analysis of the products are shown in Table I. The selectivity of MDPM was 63%. The spent oxide can be regenerated by heating at 250-600°C.

Examples 5-14

These examples were carried out under the same experimental conditions as Example 4 but using the reactant-catalysts indicated in Table I. The results are shown in Table I.

Examples 15-16

These examples were carried out under the same experimental conditions as Example 4, but Example 15 used $SnO_2$ as a support, and Example 16 used $TiO_2$ as a support. The reactant-catalyst and results are given in Table I.

Table I

| Example | Reactant-Catalyst | 1 | 2 | 3 | 4 |
|---|---|---|---|---|---|
| 4 | $V_2O_5$ | 139.0 | 29.0 | 6.6 | 63 |
| 5 | $MoO_3$ | 11.0 | 3.8 | --- | 81 |
| 6 | $Re_2O_7$ | 27.0 | 8.3 | 1.2 | 68 |
| 7 | $WO_3$ | 26.0 | --- | --- | 45 |
| 8 | $La_2O_3$ | 0.3 | --- | --- | 90 |
| 9 | $PbV_2O_6$ | 13.0 | 44.0 | --- | 51 |
| 10 | $MgMoO_4$ | 3.0 | --- | --- | 99 |
| 11 | $CoMoO_4$ | --- | 49.0 | --- | 47 |
| 12 | $PbMoO_4$ | 4.0 | --- | --- | 95 |
| 13 | $La_2(MoO_4)_3$ | 1.0 | --- | --- | 99 |
| 14 | $H_3PMo_{12}O_{40}$ | 67.0 | 0.8 | 0.6 | 59 |
| 15 | $V_2O_5/SnO_2$ | 98.0 | 29.0 | 2.8 | 61 |
| 16 | $H_6V_{10}O_{25}/TiO_2$ | --- | 8.6 | 3.7 | 72 |

In Table I, column 1 is the number of mg of toluene converted/g of catalyst; column 2 is % of benzaldehyde; column 3 is the % of benzoic acid; and column 4 is % of MDPMs.

Example 17

A sample of 450 g of p-xylene was heated with 200 g of $V_2O_5$ in a 1 liter autoclave at 260°C for 4 hrs. Nine percent of p-xylene was converted. The selectivity of (2,4',5-trimethyl)diphenyl methane is 73%.

**Claims**

1. A process for preparing a (methyl-substituted) diphenyl methane by a coupling reaction which process comprises reacting a methyl-substituted benzene with a solid heterogeneous reactant-catalyst having labile oxygen under conditions whereby the (methyl-substituted)diphenyl methane is prepared, said solid heterogeneous reactant-catalyst having labile oxygen being a compound of the formula

$M^1_aM^2_bM^3_cO_x$ (III)

wherein $M^1$ is hydrogen;
$M^2$ is other elements;
$M^3$ is at least one of V, Mo, Re, La or W;
a is 0 to 10;
b is 0 to 100;
c is 0.01 to 100; and
x is a number which satisfies the average valances of $M^1$, $M^2$ and $M^3$ in the oxidation states in which they exist in reactant-catalyst.

2. A process as claimed in Claim 1, wherein the said catalyst is selected from a vanadium oxide, a molybdenum oxide, a rhenium oxide, a tungsten oxide, and a bismuthmolybdate-containing catalyst.

3. A process as claimed in Claim 1 or Claim 2, wherein the temperature is from 50 to 700°C.

4. A process as claimed in any one of the preceding claims, wherein the selectivity to carbon dioxide is at most 65 percent.

5. A process as claimed in any one of the preceding claims, wherein the methyl-substituted benzene is

toluene or xylene.

6. A process as claimed in any one of the preceding claims, wherein the (methyl-substituted) diphenyl methane is prepared in a selectivity of at least 20 percent.

7. A process as claimed in any one of the preceding claims, which is carried out in the vapor phase.

8. A process as claimed in any one of the preceding claims, wherein an inert gaseous diluent is present.

9. A process as claimed in any one of Claims 1 to 6, which is carried out in the liquid phase.

10. A process as claimed in any one of the preceding claims, wherein the solid heterogeneous reactant-catalyst is molybdenum trioxide or vanadium pentoxide.

11. A process as claimed in any one of Claims 1 to 9, wherein the solid heterogeneous reactant-catalyst is a rhenium oxide or a tungsten oxide.

12. A process as claimed in any one of the preceding Claims, wherein the process is conducted either in the absence of oxygen or with less than the stoichiometric amount of oxygen present.

13. A process as claimed in any one of the preceding claims, wherein a support is employed.

European Patent Office

# EUROPEAN SEARCH REPORT

Application Number

EP 87 30 8069

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 151 213 (E.K. FIELDS) --- | | C 07 C 15/16<br>C 07 C 2/84 |
| A | EP-A-0 148 544 (EXXON) --- | | |
| A | US-A-4 254 293 (TREMONT et al.) ----- | | |
| | | | TECHNICAL FIELDS SEARCHED (Int. Cl.4) |
| | | | C 07 C 15/00<br>C 07 C 2/00 |

The present search report has been drawn up for all claims

| Place of search | Date of completion of the search | Examiner |
|---|---|---|
| THE HAGUE | 23-11-1987 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document